# EUROPEAN PATENT SPECIFICATION

(11) **EP 1 346 742 B1**
(45) Date of publication and mention of the grant of the patent: **09.08.2006**
(21) Application number: 03075802.3
(22) Date of filing: 19.03.2003
(51) Int. Cl.: A61M 5/32

(54) **Shieldable needle assembly with biased safety shield**
Abdeckbare Nadelvorrichtung mit angedrücktem Nadelschutz
Ensemble d'aiguille avec protecteur biasé

(30) Priority: 20.03.2002 US 365993 P
(43) Date of publication of application: 24.09.2003
(73) Proprietor: Becton Dickinson and Company, Franklin Lakes, New Jersey 07417-1880 (US)
(72) Inventor: Swenson, Kirk D., North Caldwell, NJ 07006 (US)
(74) Representative: 't Jong, Bastiaan Jacob

(56) References cited:
- EP-A- 0 433 250
- DE-A- 3 908 181
- US-A- 3 942 228
- US-A- 4 664 259
- US-A- 5 078 693
- US-A- 5 312 369
- US-A- 5 693 022
- US-B1- 6 298 541

## Description

The present invention relates to a shield for a needle and more particularly to a safety needle assembly that may be used in conjunction with a syringe assembly, a needle holder, a blood collection needle, a blood collection set, an intravenous infusion set or other fluid handling devices or assemblies that contain piercing elements.

Such a safety needle assembly as defined in the introductory part of claim 1, is known from US-A-5 693 022.

Safe and convenient handling of disposable medical devices is recognized by those in the medical arts so as to minimize exposure to blood borne pathogens. Safe and convenient handling of disposable medical devices results in the disposal of the medical devices intact.

As a result of this recognition, numerous devices have been developed for shielding needles after use. Many of these devices are somewhat complex and costly. In addition, many of these devices are cumbersome to use in performing procedures. Furthermore, some of the devices are so specific that they preclude use of the device in certain procedures or with certain devices and or assemblies.

For example, a number of devices incorporate a pivoting shield assembly in which the shield can be pivoted away from the needle during use and pivoted about the needle after use, for protection from the used needle. U.S. Patent No. 5,603,699 discloses a needle guard assembly which includes a top shield member and a bottom lever member which pivot away from the needle of a syringe in opposing directions. The assembly further includes a series of gears between the top shield member and the bottom lever member, as well as a torsional coil spring mounted there between. Such an arrangement is not practically useful due to the complex arrangement of gears and opposing pivoting members. U.S. Patent No. 5,401,251 discloses a syringe injection system including a hollow needle and a safety cap cover for covering the needle after use. The safety cap cover is attached to the body of the syringe through an elongated extension arm, which pivots the safety cap cover over the needle after use. The extension arm may be attached to the body of the syringe through a spring, which urges the arm and cover into the shielded position. Such an arrangement, however, involves extensive movement of the arm to fully expose the needle, and exposing the needle tip from the safety shield prior to use requires that the user hold the syringe in one hand and grasp the cover near the needle with the other hand, thereby potentially exposing the user to the needle tip.

In view of the foregoing, a need exists for a safety needle assembly including a shield that achieves secure and effective shielding of a used needle cannula which is simple and inexpensive to manufacture and easy to operate.

This object is achieved with the safety needle assembly according to the invention as characterized in claim 1.

A biasing element extends between the base hub and the shield, for biasing the shield toward the shielded position. The biasing element is capable of storing energy when the shield is in the non-shielded position for biasing the shield to the shielded position. The biasing element may be, for example, a leaf spring, including first and second legs at opposite ends thereof. The first leg is in engagement with the laterally extending arm of the base hub and the second leg is in engagement with the laterally extending lever of the shield. The first and second legs may form the laterally extending arm and the laterally extending lever, respectively.

The base hub and the shield may be integrally formed, desirably with the biasing element integral therebetween forming a leaf spring. The shield may include a needle cannula lock which is movable between a first position and a second position. In the first position, the needle cannula lock is bent, and provides for movement of the shield from the shielded position, such as when the needle device is in a packaged or pre-use state with the shield covering the needle cannula in a reversible shielded position. In the second position, the needle cannula lock prevents movement of the shield from the shielded position, such as when the shield has been pivotably rotated to the non-shielded position and returned to the shielded position. Such a needle cannula lock may include a finger with a needle engaging barb on one side thereof, with the needle engaging barb engaging the needle cannula for preventing pivotal movement of the shield from the shielded position when the needle cannula lock is in the second position, and with the needle cannula releasably engaging an opposite side of the finger thereby allowing for movement of the shield from the shielded position when the needle cannula lock is in the first position.

In one particular embodiment, the needle cannula lock in the first position exerts a biasing force against the needle cannula, biasing the shield toward the non-shielded position, and the biasing element exerts a biasing force greater than the biasing force exerted by the cannula lock for maintaining the shield biased toward the shielded position.

In a further embodiment, the invention is directed to a safety needle assembly for use in conjunction with a medical device such as a needle holder or a syringe. Such a safety needle assembly includes a base hub having an internal opening therethrough, and a laterally extending arm extending from the outer wall of the base hub. A needle cannula extends from a forward or distal end of the base hub, with the needle including a puncture tip and an internal lumen in communication with the internal opening of the base hub. A shield is pivotably connected to the base hub, and is pivotal with respect to the needle cannula in a similar manner as described above, including a laterally extending lever and a biasing element for pivotal movement of the shield. In such an embodiment, the base hub preferably includes structure for mating with a medical device, such as a blood collection needle holder or a syringe.

In a particular embodiment, the needle cannula of such a safety needle assembly includes a non-patient end having a non-patient puncture tip extending from one end of the base hub, and an intravenous end including an intravenous puncture tip extending from the other end of the base hub. A sleeve may extend about the non- patient puncture tip. Such a safety needle assembly is particularly adapted for mating with a blood collection needle holder through the base hub, such as through threaded engagement.

In a further embodiment, the invention relates to a safety needle assembly which includes a base hub including a proximal end and a distal end with a needle having a puncture tip extending from the distal end of the hub, a laterally extending arm adjacent the distal end of the hub and extending laterally from the hub, and a laterally extending lever extending laterally from the distal end of the hub. A hinge section is formed at the intersection of the laterally extending arm and the laterally extending lever defining an acute angle therebetween. The hinge section further defines a pivot axis for pivotal movement of the laterally extending lever with respect to the laterally extending arm between a first position and a second position with the second position defining an angle which is more acute than the first position. The assembly further includes a shield which is integral with the laterally extending lever and which extends toward the distal end of the needle for encompassing the distal end of the needle when the hinge is in the first position. As such, pivotal movement of the laterally extending lever from the first position to the second position causes biasing energy to accumulate between te laterally extending arm and the laterally extending lever, such that the biasing energy biases the laterally extending lever to the first position for encompassing the distal end of the needle.

Desirably, the biasing energy accumulates in a spring element, causing a bending moment between the laterally extending arm and the laterally extending lever. More desirably, the biasing energy accumulates in the hinge section, with the hinge section forming a leaf spring.

### DESCRIPTION OF THE DRAWINGS

FIG. 1 is a perspective view of a needle assembly attached to a needle holder not being part of the present invention, with the shield in a first packaging position prior to use;

FIG. 2 is a cross section of the needle assembly of FIG. 1;

FIG. 3 is a cross section of the needle assembly of FIG. 1 shown in a second position with the shield pivoted away from the needle for use;

FIG. 4 is a transverse cross section taken along lines 4-4 of FIG. 2, showing the cannula lock in a first packaging position prior to use;

FIG. 5 is a transverse cross section of the cannula lock in locked position when the needle assembly is in a shielded position;

FIG. 6 is a perspective view of the needle assembly of FIG. 1 shown in use in a blood collection procedure;

FIG. 7 is a perspective view of a needle assembly attached to a needle holder not being part of the present invention;

FIG. 8 is a perspective view of a needle assembly as in FIG. 1 shown with a needle cover covering the needle in an alternate packaging embodiment prior to use;

FIG. 9 is a perspective view of a double ended needle assembly for attachment to a needle holder in accordance with an embodiment of the invention;

FIG. 10 is a cross section of the needle assembly of FIG. 9 shown in a sampling position;

FIG. 11 is a cross section of the needle assembly of FIG. 9 shown in a shielded position after use; and

FIG. 12 is a cross section of a needle assembly for attachment to a syringe in accordance with a further embodiment of the present invention.

### DETAILED DESCRIPTION

While this invention is satisfied by embodiments in many different forms, there is shown in the drawings and will herein be described in detail, the preferred embodiments of the invention, with the understanding that the present disclosure is to be considered as exemplary of the principles of the invention and is not intended to limit the invention to the embodiments illustrated. Various other modifications will be apparent to and readily made by those skilled in the art without departing from the scope of the invention. The scope of the invention will be measured by the appended claims.

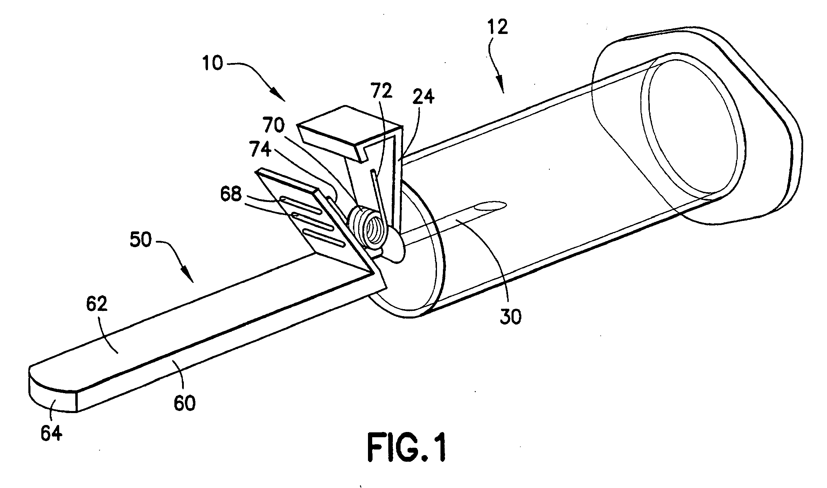

Referring to the drawings in which like reference characters refer to like parts throughout the several views thereof, FIGS. 1-3 illustrate an exemplary needle safety device not being part of the present invention and the related features, in the form of a blood collection device **10.**

The safety needle device includes a medical device, such as a needle holder **12** for use in blood collection procedures, as shown in FIGS. 1-3.

Needle holder **12** includes a generally tubular body **14** having proximal end **16** and distal end **18** at opposing ends thereof, with internal opening **20** extending therebetween. Proximal end **16** includes a flange **22**, which may extend circumferentially about proximal end **16**. Distal end **18** includes a distal opening **19** extending through tubular body **14** into internal opening **20**.

Needle holder **12** further includes a laterally extending arm **24** which extends laterally away from tubular body **14** adjacent distal end **18.** Laterally extending arm **24** extends laterally from axis **X** defining blood collection device **10,** and may be a generally planar structure. Laterally extending arm **24** is desirably integrally formed with needle holder **12**.

Blood collection device **10** further includes needle cannula **30** extending from distal end **18** of needle holder **12**. The needle cannula **30** has a proximal end **32** and an opposing distal end **34**. The needle cannula **30** defines an internal lumen **36** extending through the needle cannula **30** from proximal end **32** to distal end **34**. Distal end **34** of needle cannula **30** is beveled to define a sharp puncture tip at intravenous puncture tip **38**. Intravenous puncture tip **38** is provided for insertion into a patient's blood vessel, such as a vein, and is, therefore, designed to provide ease of insertion and minimal discomfort during venipuncture. As FIGS. 1-3 depict blood collection device **10** with needle holder **12** as a medical device for blood collection, the proximal end **32** of needle cannula **30** further includes non-patient puncture tip **40**. Non-patient puncture tip **40** is provided for puncturing of an evacuated tube, for example, during a blood collection procedure, and therefore includes a sharp puncture tip. Internal lumen **36** extends between intravenous puncture tip **38** and non-patient puncture tip **40**. An elastomeric sleeve **42** covers the non-patient puncture tip **40** at the proximal end **32**.

As noted above, while such a description relates to a medical device in the form of a needle holder for blood collection, other medical devices for use with a needle may be provided through the present invention, including a syringe.

Blood collection device **10** further includes a shield **50** pivotably connected to needle holder **12** at distal end **18**. Shield **50** comprises a rearward end **52** and a forward end **54**. Forward end **54** of shield **50** includes a slot or longitudinal opening **56** formed by sidewalls **58** and **60** that extend downwardly from top section **62** and run substantially opposite of one another in parallel along the length of slot **56** towards forward endwall **64**. Shield **50** further includes a laterally extending lever **66** which extends laterally away from top section **62** of shield **50** at rearward end **52**, adjacent distal end **18** of needle holder **12**. Laterally extending lever **66** extends laterally away from axis **X** defining the blood collection device **10,** and may be a generally planar structure integrally formed with shield **50.** Bumps or ribs **68** may be provided on a surface of laterally extending lever **66** for providing a tactile surface for engagement with a user's finger.

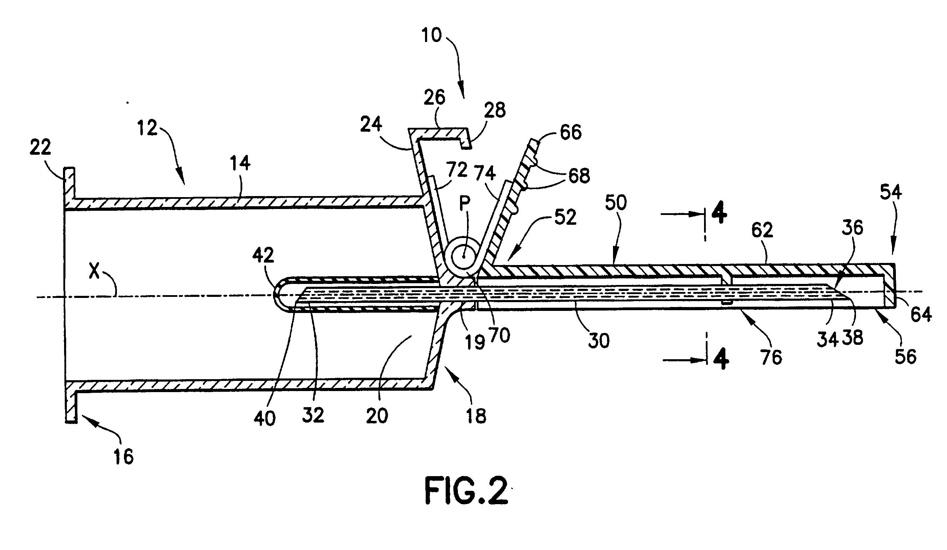

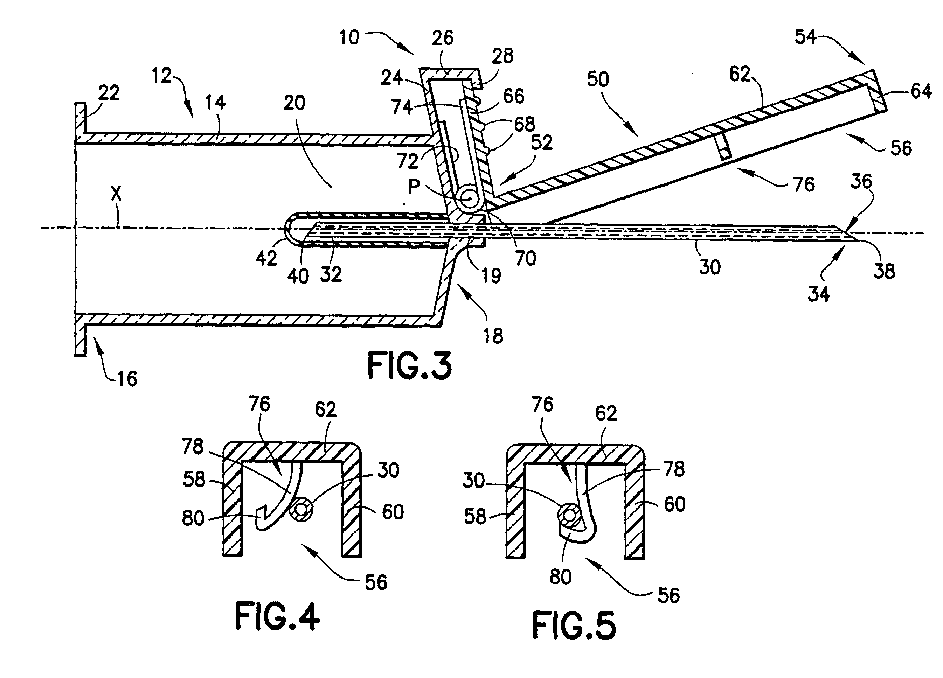

Shield **50** is pivotal with respect to needle cannula **30** about a pivoting point **P** between a retracted or non-shielded position as shown in FIG. 3 in which shield **50** is pivotally spaced from distal end **34** of needle cannula **30**, and a shielded position as shown in FIG. 2 in which the distal end **34** of needle cannula **30** is encompassed within slot **56** of shield **50**.

Blood collection device **10** further includes a biasing element, such as spring **70**, extending between laterally extending arm **24** of needle holder **12** and laterally extending lever **66** of shield **50**. Spring **70** provides a biasing force between needle holder **12** and shield **50**, and includes stored energy for biasing shield **50** toward the shielded position encompassing needle cannula **30**. Spring **70** may be a wound torsion spring such as a coil spring shown in FIGS. 1-3, a compression spring, or a leaf spring.

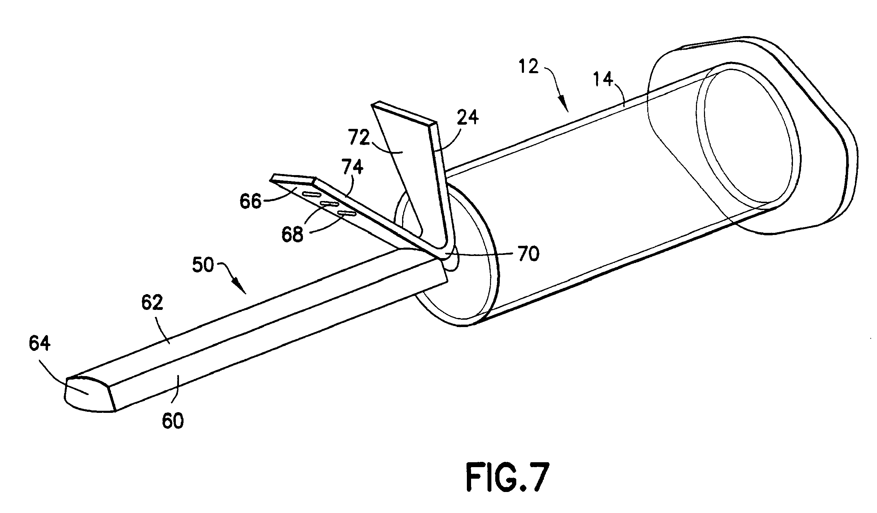

Spring **70** includes a first leg **72** and a second leg **74** at opposing ends. First leg **72** is in engagement with laterally extending arm **24** of needle holder **12**, and second leg **74** is in engagement with laterally extending lever **66** of shield **50**. As shown in FIGS. 1-3, such engagement may be achieved with first leg 72 and second leg **74** lying against and/or fixedly adhered to the planar surfaces of laterally extending arm **24** and laterally extending lever **66**, respectively. Alternatively, first leg **72** and second leg **74** of spring **70** may comprise laterally extending arm **24** and laterally extending lever **66**, such as depicted in FIG. 7, with first leg **72** and second leg **74** of the leaf spring forming laterally extending arm **24** and laterally extending lever **66**, respectively. As such, laterally extending arm **24** and laterally extending lever **66** intersect to form a hinge section at spring **70**, with shield **50** integral with the laterally extending lever **66** and extending toward the distal end of needle cannula **30**. The leaf spring acts as a biased hinge between laterally extending arm **24** and laterally extending lever **66.** In such a case, needle holder **12,** shield **50** and spring **70** are preferably unitarily formed as an integral part.

Laterally extending arm **24** and laterally extending lever **66** extend away from the same side of a plane defined by the longitudinal axis **X** of blood collection device **10** and the pivoting point **P** of shield **50.** Moreover, first leg **72** and second leg **74** of spring **70** also extend away from the same side of a plane defined by the longitudinal axis **X** of blood collection device **10** and the pivoting point **P** of shield **50.** Such an arrangement provides first leg **72** and second leg **74,** and therefore laterally extending arm **24** and laterally extending lever 66 in engagement therewith, in close approximation with one another for ease of movement therebetween, as will be described in more detail herein.

Desirably, laterally extending arm **24** of needle holder **12** and laterally extending lever **66** of shield **50** include interengaging structure for releasably holding shield **50** in the retracted position. Such interengaging structure may be provided through a latch mechanism, such as by providing laterally extending arm **24** with a planar surface **26** and a latch **28**, for releasably engaging the top edge of laterally extending lever **66** of shield **50**, as shown in FIG. 3. It is contemplated that other releasable engaging arrangements may be used, for example, by providing laterally extending lever **66** with such a latching mechanism for engagement with laterally extending arm **24**.

Shield **50** may include means for trapping the needle cannula **30** in slot **56**, such as a needle cannula lock **76**. As shown in FIGS. 4 and 5, such needle cannula lock **76** includes a finger **78** that extends from an interior portion of top section **62**, with a needle engaging barb **80** extending from one side thereof. Finger **78** of needle cannula lock **76** is a resiliently flexible material. The needle cannula lock **76** is movable between a first position shown in FIG. 4, permitting pivotal movement of shield **50**, and a second position shown in FIG. 5, preventing pivotal movement of shield **50**. More particularly, when shield **50** is in a first position, such as during packaging prior to use, finger **78** of needle cannula lock **76** is in a first bent position, with needle cannula **30** sitting against one side of finger **78**. The resilient flexible nature of finger **78** exerts a biasing force against needle cannula **30** with finger **78** in this first position, biasing shield **50** toward the retracted position. Spring **50**, however, exerts a biasing force in the opposing direction biasing the shield toward the shielded position, which biasing force of spring **50** is greater than the biasing force of finger **78** in this first position, thereby maintaining the shield biased toward the shielded position, for packaging. The resilient nature of finger **78** causes finger **78** to move from the first bent position to a second relaxed or rest position when needle cannula **30** is out of engagement therewith, such as when shield **50** is pivoted to the retracted position. When shield **50** is again pivoted to the shielded position, such as when a procedure is completed, finger **78** slightly deflects to the opposing side of finger **78**, whereby the needle is permanently trapped by needle engaging barb **80**. Such an arrangement provides a needle cannula lock which is automatically movable between a first position, which permits movement of shield **50** to a retracted position, and a second position, which prevents movement of shield **50** from the shielded position. It is further contemplated that the needle cannula lock may include a mechanism for mechanically engaging the lock when shield **50** is in a retracted position.

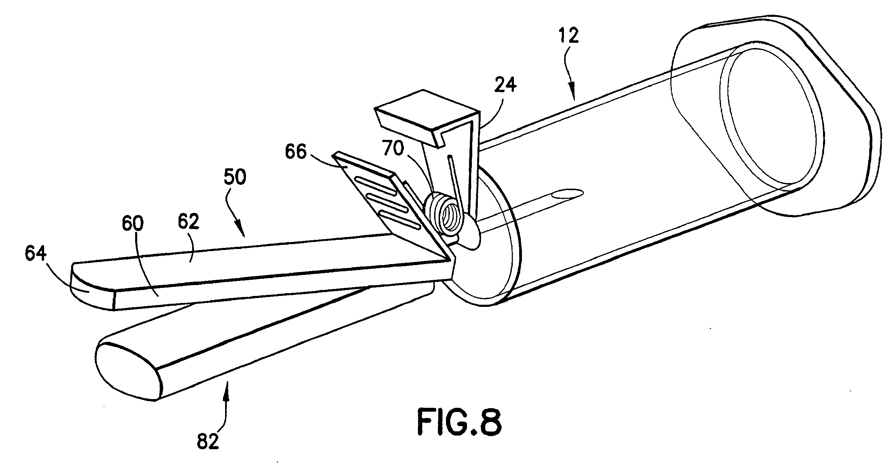

As noted, such a needle cannula lock may provide blood collection device **12** in a one time reversible shielded position during packaging. A removable protective cover may further be provided along slot **56** in this packaged condition. Alternatively, as depicted in FIG. 8, shield **50** may be slightly retracted during packaging, with a removable protective needle cover such as rigid sleeve **82** positioned over distal end **34** of needle cannula **30** for protection from intravenous puncture tip **38** during packaging and prior to use.

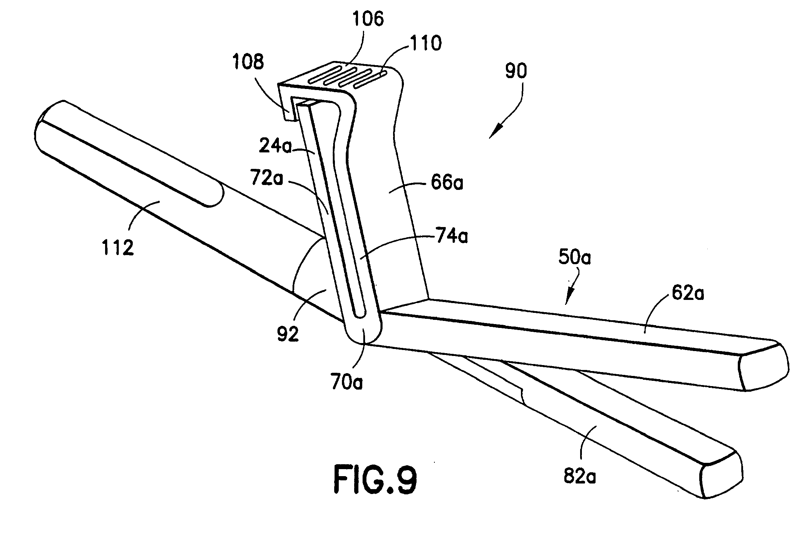

FIGS. 9-12 depict an embodiment of the invention that includes many components which are substantially identical to the components of FIGS. 1-8. Accordingly, similar components performing similar functions will be numbered identically to those components of FIGS. 1-8, except that a suffix "a" will be used to identify those similar components in FIGS. 9-12.

The embodiment of FIGS. 9-12 includes safety needle assembly **90** for use with conventional medical devices, such as conventional needle holders for blood collection, syringes, and the like. As shown in FIGS. 9-12, the safety needle assembly **90** includes a needle cannula **30a**, a shield **50a** and a biasing element in the form of spring **70a**, as set forth in the embodiment described above. In the embodiment of FIGS. 9-12, the safety needle assembly **90** is an independent component for attachment to a medical device, and further includes a base hub **92** for providing such attachment.

Base hub **92** includes a proximal end **94** and distal end **96,** with an internal opening **98** extending therethrough. Needle cannula **30a** extends through internal opening **98** of base hub **92**, with proximal end **32a** of needle cannula **30a** extending from proximal end **94** of base hub **92**, and distal end **34a** of needle cannula **30a** extending from distal end **96** of base hub **92**. Base hub **92** further includes laterally extending arm **24a** which extends laterally away from base hub **92** adjacent distal end **96**, in a similar manner as with laterally extending arm **24** described above in connection with the device of FIGS. 1-8.

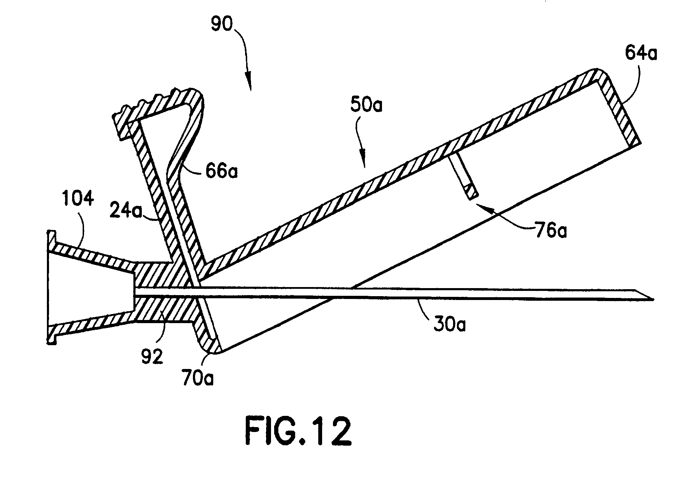

Base hub **92** may include a threaded end **100** at the proximal end thereof. Preferably, threaded end **100** comprises male threads **102** for mounting the hub on a conventional needle holder. Alternatively, as shown in FIG. 12, base hub **92** may include a female luer fitting **104** at the proximal end thereof for attachment with a male luer fitting, and may include additional luer lugs for attachment with a luer collar, such as a syringe luer collar.

Base hub **92** is interconnected with shield **50a** in a similar manner as the interconnection between needle holder **12** and shield **50** in the previously described device of FIGS. 1-8. Desirably, base hub **92** and shield **50a** are integrally formed. Spring **70a** is provided between base hub **92** and shield **50a**, and is desirably a leaf spring, with first leg **72a** and second leg **74a** forming laterally extending arm **24a** of base hub **92** and laterally extending lever **66a** of shield **50a**. In such an embodiment, interengaging structure between laterally extending arm **24a** of base hub **92** and laterally extending lever **66a** of shield **50a** is desirably provided through lever planar surface **106** and latch **108** of laterally extending lever **66a**, which engage a top edge of laterally extending arm **24a**. Lever planar surface **106** preferably includes ribs **110** as a tactile surface for a user.

Safety needle assembly **90** may be packaged with a removable protective cover provided along slot **56a** as discussed above, or, as depicted in FIG. 9, shield **50a** may be slightly retracted during packaging, with a removable protective needle cover such as rigid sleeve **82a** positioned over distal end **34a** of needle cannula **30a** for protection from intravenous puncture tip **38a** during packaging and prior to use. In addition, a second rigid sleeve **112** may be provided over proximal end **32a** of needle cannula **30a** for protection from non-patient puncture tip **40a**.

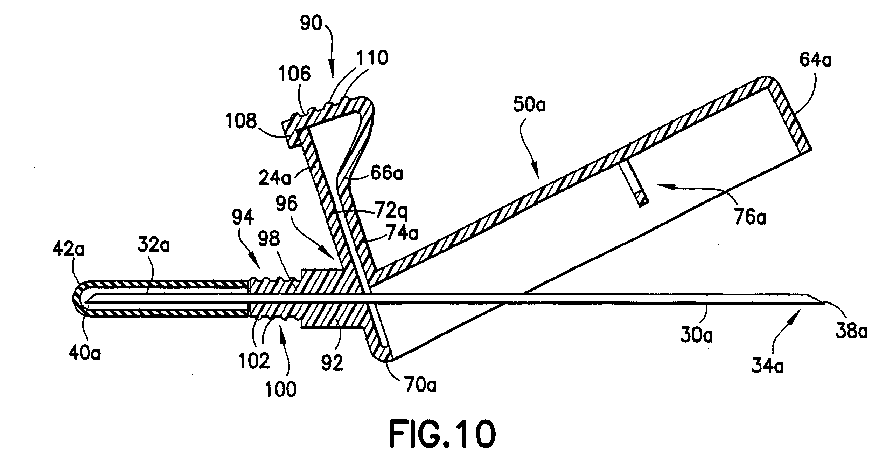

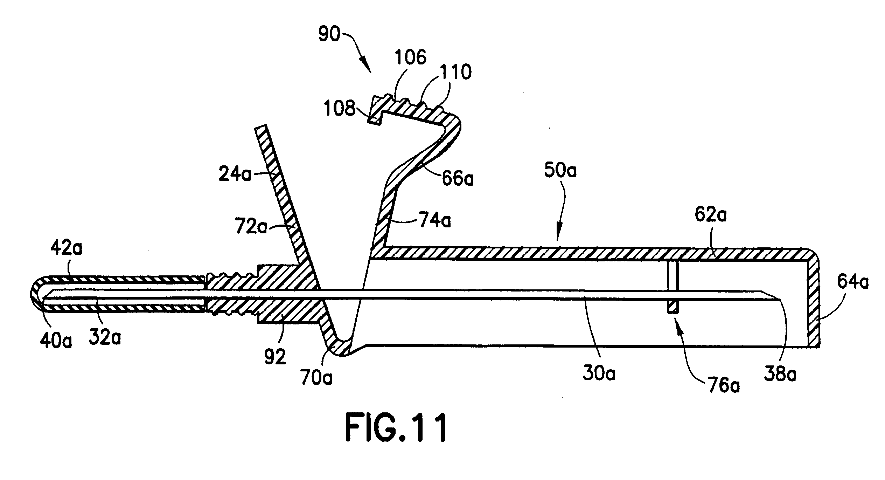

With the basic structure of the assembly now described, operation of a needle safety device will be described with reference to the blood collection device **10** shown in FIGS. 1-7. In use, the blood collection device **10** is provided as depicted in FIG. 1, with shield **50** in a shielded position and with finger **78** of the needle cannula lock **76** in a first bent position as in FIG. 4. A protective covering may be provided over slot **56**, which protective covering is removed in preparation for use. To prepare for use of the blood collection device **10**, the user applies a force between laterally extending arm **24** and laterally extending lever **66**, such as by pinching them together between the user's fingers, thereby causing shield **50** to pivot about the pivoting point **P** to a retracted or non-shielded position. This pinching causes the hinge section between laterally extending arm **24** and laterally extending lever **66** to move from a first position defining an acute angle as shown in FIGS. 2 and 11, to a second position defining an acute angle which is more acute that the angle defined by this hinged section in the first position, as shown in FIGS. 3 and 10. Such pivoting causes biasing energy to accumulate between laterally extending arm **24** and laterally extending lever **66** and be stored in the biasing element of spring **70**, thereby causing a bending moment between laterally extending arm **24** and laterally extending lever **66**. Such pivoting also causes finger **78** of needle cannula lock **76** to move from the first bent position to a second relaxed position. The top edge of laterally extending lever **66** is engaged by latch **28** of laterally extending arm **24**, thereby locking shield **50** in the retracted position with intravenous puncture tip **38** exposed for use. In devices including a rigid sleeve **82** as a packaging cover over the distal end of the needle cannula as depicted in FIG. 8, the user grasps the assembly in one hand and applies a pinching force in a similar manner as described above. With the other hand, the user removes the rigid sleeve **82** from needle cannula **30**, thereby exposing intravenous puncture tip **38** for use.

The medical practitioner then sterilizes the intended area of puncture on the patient's body, and can then urge intravenous puncture tip **38** at distal end **34** of needle cannula **30** into a targeted blood vessel of a patient. An appropriate medical procedure can then be conducted. Upon completion of the procedure, such as when all desired samples have been drawn, needle cannula **30** is withdrawn from the patient, and shielding of the needle can be accomplished. In particular, the user lifts latch **28** to release the top edge of laterally extending lever **66**, thereby releasing the interengagement with shield **50**. The stored energy of spring **70** causes shield **50** to pivot about pivoting point **P** to the shielded position. Hence, shield **50** safely shields and encompasses needle cannula **30** and intravenous puncture tip **38**. In addition, needle engaging barb **80** of needle cannula lock **76** engages needle cannula **30**, as shown in FIG. 5, thereby preventing any further pivotal movement of shield **50** to the retracted position. The needle safety device can then be safely discarded.

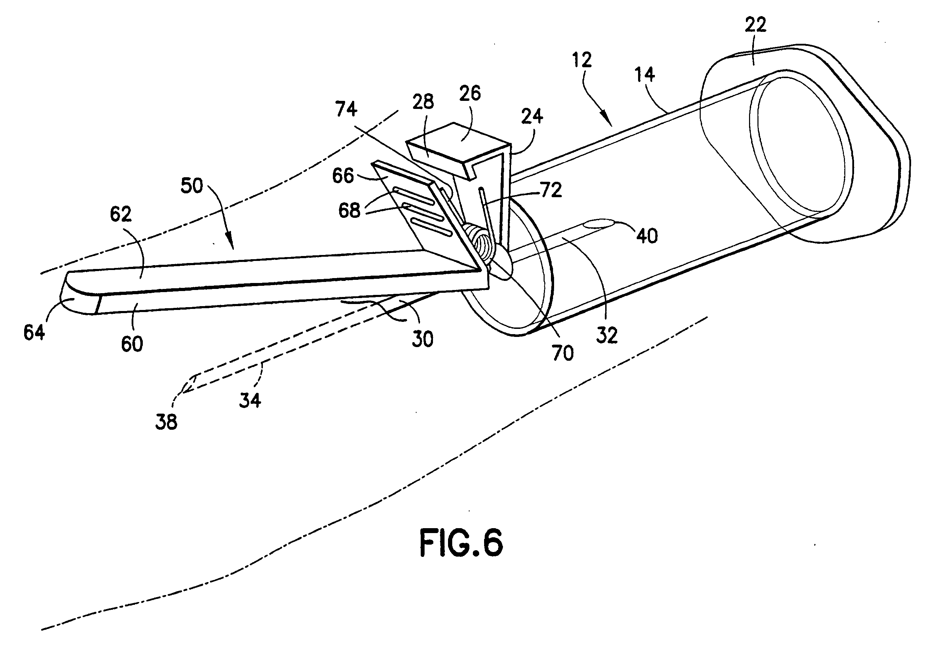

Shielding of the needle may also be passively accomplished. In particular, it is noted that activation of the safety shield may be accomplished while venipucture is maintained, that is while intravenous puncture tip **38** of needle cannula **30** is maintained within the blood vessel of the patient. For example, once intravenous puncture tip **38** of the needle cannula **30** is inserted into a blood vessel in the patient's body (i.e., venipuncture), the user can lift latch **28** to release the top edge of laterally extending lever **66**, thereby releasing the interengagement with shield **50**, and causing shield **50** to pivot around pivoting point **P** due to the biasing force of spring **70**. Since intravenous puncture tip **38** is within the patient's blood vessel, such pivotal movement of shield **50** will terminate when the forward end **54** of shield **50** contacts the skin of the patient, as shown in FIG. 6. Upon removal of intravenous puncture tip **38** from the patient's blood vessel, shield **50** will continue in its pivotal rotation to the shielded state, thereby shielding intravenous puncture tip **50** and needle cannula **30** and locking needle cannula lock **76** in place.

In the embodiment of FIGS. 9-12, the safety needle assembly **90** is assembled with an appropriate medical device, such as a needle holder, prior to use. For example, second rigid sleeve **112** is removed, and the needle holder is screwed onto base hub **92** through threads **102**. The user then removes rigid sleeve **82a** from distal end **34a** of needle cannula **30a** in a similar manner as described above, thereby exposing intravenous puncture tip **38a** for use, and pivoting and locking shield **50a** in the retracted position. The safety needle assembly can then be used for an appropriate medical procedure and the shield **50a** can thereafter be pivoted to the shielded position, as discussed above.

The shield and hub of the safety shield assembly of the present invention are comprised of moldable parts which can be mass produced from a variety of materials including, for example, polyethylene, polyvinyl chloride, polystyrene or polyethylene and the like. Materials will be selected which will provide the proper covering and support for the structure of the invention in its use, but which will provide also a degree of resiliency for the purpose of providing the cooperative movement relative to the shield and the hub of the assembly.

While the needle assembly of the present invention has been described in terms of one embodiment for use in connection with a blood collection system, it is further contemplated that the needle assembly could be used with other medical procedures, such as in conjunction with conventional intravenous infusion sets, which are well known in the art for use with needle assemblies.

## Claims

1. A safety needle assembly (90) comprising:
a base hub (92)
a needle cannula (30a) extending from a distal end of said base hub, said needle cannula including a distal end having a puncture tip (38a);
a shield (50a) pivotably connected to said base hub, said shield being pivotal with respect to said needle cannula between a shielded position encompassing said distal end of said needle cannula and a non-shielded position pivotally spaced from said distal end of said needle cannula, said shield including a laterally extending lever (66a) extending away from a plane defined by the longitudinal axis of said needle cannula and a pivot of said shield; and
a biasing element (70a) extending between said hub and said shield for biasing said shield toward the shielded position, said biasing element capable of storing energy when said shield is in said non-shielded position for biasing said shield to said shielded position, wherein said base hub includes a laterally extending arm (24a), said laterally extending lever and said laterally extending arm extending adjacent one another and away from the same side of the plane defined by the longitudinal axis of said needle cannula and the pivot of said shield, said biasing element extending between said laterally extending arm of said hub and said laterally extending lever, **characterized by** said laterally extending lever of said shield and said laterally extending arm of said hub including an interengaging structure (108) or releasably holding said shield in said non-shielded position.

2. A safety needle assembly as in claim 1, wherein said biasing element comprises a spring extending between said hub and said shield for biasing said shield toward the shielded position, said spring including first and second legs at opposite ends thereof, said first and second legs extending away from the same side of a plane defined by the longitudinal axis of said needle cannula and a pivot of said shield, said first leg in engagement with said laterally extending arm of said hub and said second leg in engagement with said laterally extending lever of said shield.

3. A safety needle assembly as in claim 1, wherein said biasing element is a leaf spring.

4. A safety needle assembly as in claim 3, wherein said hub and said shield are integral, a first leg of said leaf spring comprises said laterally extending arm of said hub and a second leg of said leaf spring comprises said laterally extending lever of said shield.

5. A safety needle assembly as in any of claims 1-4, wherein said shield includes a needle cannula lock which in a first position will provide for movement of said shield from said shielded position and in a second position will prevent movement of said shield from said shielded position.

6. A safety needle assembly as in claim 5 , wherein said needle cannula lock in said first position exerts a biasing force against said needle cannula biasing said shield toward the non-shielded position, and said biasing element exerts a biasing force greater than said biasing force exerted by said cannula lock for maintaining said shield biased toward the shielded position.

7. A safety needle assembly as in claim 5 or 6, wherein said needle cannula lock is automatically movable from said first position to said second position.

8. A safety needle assembly as in any of claims 5-7, wherein said needle cannula lock includes a finger with a needle engaging barb on one side thereof, and wherein with said needle cannula lock in said second position said barb engages said needle cannula preventing pivotal movement of said shield from said shielded position and in said first position said needle cannula releasably engages an opposite side of said finger allowing for movement of said shield from said shielded position.

9. A safety needle assembly as in any of claims 1-8, wherein said hub includes an internal opening therethrough and said needle cannula includes an internal lumen in fluid communication with said internal opening of said hub, and wherein said needle cannula extends through said internal opening of said hub, and includes a proximal end extending from a proximal end of said hub and including a non-patient puncture tip, and a distal end extending from a distal end of said hub and including an intravenous puncture tip.

10. A safety needle assembly as in any of claims 1-9, wherein said hub includes structure for mating with a medical device.

11. A safety needle assembly as in claim 1, wherein said pivotal connection of said shield and said base hub is being provided by a hinge section formed at the intersection of said laterally extending arm and said laterally extending lever and defining an acute angle therebetween;
said hinge section further defining a pivot axis for pivotal movement of said laterally extending lever with respect to said laterally extending arm between a first position and a second position with said second position defining an angle which is more acute than said first position;
said biasing energy being accumulated between said laterally extending arm and said laterally extending lever.

12. A safety needle assembly as in claim 11, wherein said biasing energy accumulates in a spring element causing a bending moment between said laterally extending arm and said laterally extending lever.

13. A safety needle assembly as in claim 11, wherein said biasing energy accumulates in the hinge section, said hinge section comprising a leaf spring.

## Patentansprüche

1. Nadelsicherheitsvorrichtung (90), umfassend:
einen Grundkörper (92);
eine Nadelkanüle (30a), die sich von einem distalen Ende des Grundkörpers erstreckt, wobei die Nadelkanüle ein distales Ende mit einer Punktierspitze (38a) aufweist;
einen Schutz (50a), der schwenkbar mit dem Grundkörper verbunden ist, wobei der Schutz in Bezug auf die Nadelkanüle verschwenkbar ist zwischen einer schützenden Stellung, in der er das distale Ende der Nadelkanüle umfaßt und einer nicht schützenden Stellung, in der er von dem distalen Ende der Nadelkanüle verschwenkt beabstandet ist, wobei der Schutz einen seitlich verlaufenden Arm (66a) aufweist, der sich von einer Ebene weg erstreckt, die definiert ist durch die Längsachse der Nadelkanüle und einen Drehpunkt des Schutzes; und
ein Vorspannelement (70a), das sich zwischen dem Körper und dem Schutz erstreckt, um den Schutz in Richtung der schützenden Stellung vorzuspannen, wobei das Vorspannelement fähig ist, Energie zu speichern, wenn sich der Schutz in der nicht schützenden Stellung befindet, um den Schutz in die schützende Stellung vorzuspannen, wobei der Grundkörper einen sich seitlich erstreckenden Arm (24a) aufweist, wobei der seitlich verlaufende Arm und der sich seitlich erstreckende Arm aneinander angrenzend verlaufen und von der gleichen Seite der Ebene weg, die definiert ist durch die Längsachse der Nadelkanüle und den Drehpunkt des Schutzes, wobei das Vorspannelement sich zwischen dem sich seitlich erstreckenden Arm des Körpers und dem seitlich verlaufenden Arm erstreckt, **dadurch gekennzeichnet, daß** der seitlich verlaufende Arm des Schutzes und der sich seitlich erstreckende Arm des Körpers eine Eingriffsstruktur (108) aufweisen, um den Schutz in der nicht schützenden Stellung lösbar zu halten.

2. Nadelsicherheitsvorrichtung nach Anspruch 1, bei welcher das Vorspannelement eine Feder umfaßt, die zwischen dem Körper und dem Schutz verläuft, um den Schutz in Richtung der schützenden Stellung vorzuspannen, wobei die Feder erste und zweite Schenkel an ihren gegenüberliegenden Enden aufweist, wobei die ersten und zweiten Schenkel von der gleichen Seite einer Ebene weg verlaufen, die definiert ist durch die Längsachse der Nadelkanüle und einen Drehpunkt des Schutzes, wobei der erste Schenkel in Eingriff steht mit dem sich seitlich erstreckenden Arm des Körpers und der zweite Schenkel in Eingriff steht mit dem seitlich verlaufenden Arm des Schutzes.

3. Nadelsicherheitsvorrichtung nach Anspruch 1, bei welcher das Vorspannelement eine Blattfeder ist.

4. Nadelsicherheitsvorrichtung nach Anspruch 3, bei welcher der Körper und der Schutz integral sind, ein erster Schenkel der Blattfeder den sich seitlich erstreckenden Arm des Körpers umfaßt und ein zweiter Schenkel der Blattfeder den seitlich verlaufenden Arm des Schutzes umfaßt.

5. Nadelsicherheitsvorrichtung nach einem der Ansprüche 1-4, bei welcher der Schutz eine Nadelkanülensperre aufweist, die in einer ersten Stellung eine Bewegung des Schutzes aus der schützenden Stellung vorsieht und in einer zweiten Stellung eine Bewegung des Schutzes aus der schützenden Stellung verhindert.

6. Nadelsicherheitsvorrichtung nach Anspruch 5, bei welcher die Nadelkanülensperre in der ersten Stellung eine Vorspannkraft gegen die Nadelkanüle ausübt, durch welche der Schutz in Richtung der nicht schützenden Stellung vorgespannt wird und das Vorspannelement eine Vorspannkraft ausübt, die größer ist als die von der Kanülensperre ausgeübte Vorspannkraft, um den Schutz in Richtung der schützenden Stellung vorgespannt zu halten.

7. Nadelsicherheitsvorrichtung nach Anspruch 5 oder 6, bei welcher die Nadelkanülensperre automatisch von der ersten Stellung in die zweite Stellung bewegbar ist.

8. Nadelsicherheitsvorrichtung nach einem der Ansprüche 5-7, bei welcher die Nadelkanülensperre einen Finger mit einem Nadeleingriffswiderhaken an einer ihrer Seiten aufweist und wobei, wenn sich die Nadelkanülensperre in der zweiten Stellung befindet, der Widerhaken in Eingriff steht mit der Nadelkanüle und eine Schwenkbewegung des Schutzes aus der schützenden Stellung verhindert und wenn sie sich in der ersten Stellung befindet, die Nadelkanüle lösbar in Eingriff steht mit einer gegenüberliegenden Seite des Fingers und eine Bewegung des Schutzes aus der schützenden Stellung erlaubt.

9. Nadelsicherheitsvorrichtung nach einem der Ansprüche 1-8, bei welcher der Körper eine durchgehende innere Öffnung aufweist und die Nadelkanüle ein inneres Lumen aufweist, das in fluidmäßiger Verbindung steht mit der inneren Öffnung des Körpers und wobei die Nadelkanüle durch die innere Öffnung des Körpers verläuft und ein proximales Ende aufweist, das sich von einem proximalen Ende des Körpers erstreckt und eine nicht patientenmäßige Punktierspitze aufweist, sowie ein distales Ende, das sich von einem distalen Ende des Körpers erstreckt und eine intravenöse Punktierspitze aufweist.

10. Nadelsicherheitsvorrichtung nach einem der Ansprüche 1-9, wobei der Körper eine Struktur aufweist, um mit einer medizinischen Vorrichtung zusammenzupassen.

11. Nadelsicherheitsvorrichtung nach Anspruch 1, bei welcher die Schwenkverbindung des Schutzes und des Grundkörpers vorgesehen wird durch einen Gelenkabschnitt, der ausgebildet ist bei der Schnittstelle des seitlich sich erstreckenden Armes und des seitlich verlaufenden Armes und einen spitzen Winkel dazwischen definiert;
der Gelenkabschnitt des weiteren eine Schwenkachse definiert für die Schwenkbewegung des seitlich verlaufenden Armes in Bezug auf den sich seitlich erstreckenden Arm zwischen einer ersten Stellung und einer zweiten Stellung, wobei die zweite Stellung einen Winkel definiert, der spitzer ist als die erste Stellung;
die Vorspannenergie aufgespeichert wird zwischen dem sich seitlich erstreckenden Arm und dem seitlich verlaufenden Arm.

12. Nadelsicherheitsvorrichtung nach Anspruch 11, bei welcher die Vorspannenergie in einem Federelement gespeichert wird, wodurch ein Biegemoment zwischen dem sich seitlich erstreckenden Arm und dem seitlich verlaufenden Arm bewirkt wird.

13. Nadelsicherheitsvorrichtung nach Anspruch 11, bei welcher die Vorspannenergie in dem Gelenkabschnitt gespeichert wird, wobei der Gelenkabschnitt eine Blattfeder umfaßt.

## Revendications

1. Ensemble d'aiguille de sécurité (90) comprenant :
un raccord de base (92) ;
une canule d'aiguille (30a) s'étendant à partir d'une extrémité distale dudit raccord de base, ladite canule d'aiguille comprenant une extrémité distale ayant une pointe de perforation (38a) ;
une protection (50a) reliée par pivotement audit raccord de base, ladite protection pouvant pivoter par rapport à ladite canule d'aiguille entre une position protégée englobant ladite extrémité distale de ladite canule d'aiguille et une position non protégée espacée par pivotement de ladite extrémité distale de ladite canule d'aiguille, ladite protection comprenant un levier (66a) qui s'étend latéralement à distance d'un plan défini par l'axe longitudinal de ladite canule d'aiguille et un pivot de ladite protection ; et
un élément de sollicitation (70a) s'étendant entre ledit raccord et ladite protection afin de pousser ladite protection vers la position protégée, ledit élément de sollicitation étant capable de stocker l'énergie lorsque ladite protection se trouve dans ladite position non protégée afin de pousser ladite protection vers ladite position protégée ; dans lequel :
ledit raccord de base comprend un bras (24a) qui s'étend latéralement, ledit levier s'étendant latéralement et ledit bras s'étendant latéralement s'étendant de manière adjacente l'un à l'autre et à distance du même côté du plan défini par l'axe longitudinal de ladite canule d'aiguille et le pivot de ladite protection, ledit élément de sollicitation s'étendant entre ledit bras s'étendant latéralement dudit raccord et ledit levier s'étendant latéralement, **caractérisé en ce que** ledit levier s'étendant latéralement de ladite protection et ledit bras s'étendant latéralement dudit raccord comprennent une structure de mise en prise mutuelle (107) destinée à maintenir de manière détachable ladite protection dans ladite position non protégée.

2. Ensemble d'aiguille de sécurité selon la revendication 1, dans lequel ledit élément de sollicitation comprend un ressort s'étendant entre ledit raccord et ladite protection afin de pousser ladite protection vers la position protégée, ledit ressort comprenant une première et une seconde jambes au niveau de ses extrémités opposées, ladite première et seconde jambes s'étendant à distance du même côté d'un plan défini par l'axe longitudinal de ladite canule d'aiguille et un pivot de ladite protection, ladite première jambe étant en prise avec ledit bras s'étendant latéralement dudit raccord et ladite seconde jambe étant en prise avec ledit levier s'étendant latéralement de ladite protection.

3. Ensemble d'aiguille de sécurité selon la revendication 1, dans lequel ledit élément de sollicitation est un ressort à lames.

4. Ensemble d'aiguille de sécurité selon la revendication 3, dans lequel ledit raccord et ladite protection sont solidaires, une première jambe dudit ressort à lames comprend ledit bras s'étendant latéralement dudit raccord et une seconde jambe dudit ressort à lames comprend ledit levier s'étendant latéralement de ladite protection.

5. Ensemble d'aiguille de sécurité selon l'une quelconque des revendications 1 à 4, dans lequel ladite protection comprend un dispositif de verrouillage de canule d'aiguille qui, dans une première position, assure le mouvement de ladite protection à partir de ladite position protégée et, dans une seconde position, empêche le mouvement de ladite protection à partir de ladite position protégée.

6. Ensemble d'aiguille de sécurité selon la revendication 5, dans lequel ledit dispositif de verrouillage de canule d'aiguille, dans ladite première position, exerce une force de poussée contre ladite canule d'aiguille poussant ladite protection vers la position non protégée, et ledit élément de sollicitation exerce une force de poussée supérieure à ladite force de poussée exercée par ledit dispositif de verrouillage de canule afin de maintenir ladite protection poussée vers la position protégée.

7. Ensemble d'aiguille de sécurité selon la revendication 5 ou 6, dans lequel ledit dispositif de verrouillage de canule d'aiguille peut être automatiquement déplacé à partir de ladite première position dans ladite seconde position.

8. Ensemble d'aiguille de sécurité selon l'une quelconque des revendications 5 à 7, dans lequel ledit dispositif de verrouillage de canule d'aiguille comprend un doigt avec une barbelure de mise en prise d'aiguille d'un côté de celui-ci et dans lequel, avec ledit dispositif de verrouillage de canule d'aiguille dans ladite seconde position, ladite barbelure met en prise ladite canule d'aiguille en empêchant le mouvement pivotant de ladite protection à partir de ladite position protégée et, dans ladite première position, ladite canule d'aiguille met en prise de manière détachable un côté opposé dudit doigt, ce qui permet le mouvement de ladite protection à partir de ladite position protégée.

9. Ensemble d'aiguille de sécurité selon l'une quelconque des revendications 1 à 8, dans lequel ledit raccord comprend une ouverture interne qui le traverse et ladite canule d'aiguille comprend une lumière interne en communication fluidique avec ladite ouverture interne dudit raccord, et dans lequel ladite canule d'aiguille s'étend à travers ladite ouverture interne dudit raccord, et comprend une extrémité proximale s'étendant à partir d'une extrémité proximale dudit raccord et comprenant une pointe de perforation qui n'est pas destinée au patient, et une extrémité distale s'étendant à partir d'une extrémité distale dudit raccord et comprenant une pointe de perforation intraveineuse.

10. Ensemble d'aiguille de sécurité selon l'une quelconque des revendications 1 à 9, dans lequel ledit raccord comprend une structure destinée à s'accoupler à un dispositif médical.

11. Ensemble d'aiguille de sécurité selon la revendication 1, dans lequel ledit raccordement par pivotement de ladite protection et dudit raccord de base est fourni par une section de charnière formée au niveau de l'intersection dudit bras s'étendant latéralement et dudit levier s'étendant latéralement et définissant un angle aigu entre ceux-ci ;
ladite section de charnière définit en outre un axe de pivotement pour le mouvement pivotant dudit levier s'étendant latéralement par rapport audit bras s'étendant latéralement entre une première position et une seconde position, ladite seconde position définissant un angle qui est plus aigu que ladite première position ;
ladite énergie de sollicitation est accumulée entre ledit bras s'étendant latéralement et ledit levier s'étendant latéralement.

12. Ensemble d'aiguille de sécurité selon la revendication 11, dans lequel ladite énergie de sollicitation s'accumule dans un élément de ressort qui provoque un moment de flexion entre ledit bras s'étendant latéralement et ledit levier s'étendant latéralement.

13. Ensemble d'aiguille de sécurité selon la revendication 11, dans lequel ladite énergie de sollicitation s'accumule dans la section de charnière, ladite section de charnière comprenant un ressort à lames.
